## Europäisches Patentamt
## European Patent Office
## Office européen des brevets

(19)

(11) Numéro de publication: **0 068 930**
**A2**

(12)
# DEMANDE DE BREVET EUROPEEN

(21) Numéro de dépôt: **82400988.0**

(22) Date de dépôt: **27.05.82**

(51) Int. Cl.³: **A 61 B 6/00**

(30) Priorité: **10.06.81 BE 889157**

(43) Date de publication de la demande: **05.01.83**
**Bulletin 83/1**

(84) Etats contractants désignés: **DE FR IT NL**

(71) Demandeur: **THOMSON-CSF, 173, Boulevard Haussmann, F-75379 Paris Cedex 08 (FR)**

(72) Inventeur: **Munch, Joseph, THOMSON-CSF SCPI 173, bld Haussmann, F-75379 Paris Cedex 08 (FR)**

(74) Mandataire: **Dubreuil, Annie et ai, THOMSON-CSF SCPI 173, Bld Haussmann, F-75379 Paris Cedex 08 (FR)**

(54) **Téléscope à tubes coulissants.**

(57) L'invention concerne un télescope à tubes coulissants, permettant d'ajuster la position d'un appareil qu'il supporte.

Les tubes (2, 3, 4, 5) coulissants l'un dans l'autre, sont des tubes profilés étirés, et sur leur surface intérieure sont prévues des sertissures longitudinales (7), dans lesquelles est serrée une barre (8) en acier trempé sur laquelle roulent des roulements à billes (9), montés sur la partie supérieure de la surface du manteau de chaque tube (3, 4, 5).

L'invention est applicable notamment au domaine de la radiologie.

## TELESCOPE A TUBES COULISSANTS

L'invention concerne un télescope à tubes coulissants, permettant d'ajuster la position d'un appareil qu'il supporte. Un tel appareil pouvant être par exemple un porte tube Röntgen d'un équipement Röntgen.

Jusqu'ici, on connaît un télescope qui se compose de plusieurs tubes en acier dur, coulissant l'un dans l'autre et entre lesquels il est placé des billes qui permettent de faire coulisser plus facilement les tubes l'un dans l'autre.

Ce télescope, pour la fabrication duquel il faut nécessairement utiliser de l'acier dur, présente toutefois l'inconvénient d'être relativement lourd, de sorte qu'il faut prendre des dispositions spéciales pour suspendre le télescope. Le prix de revient d'un tel télescope est en outre relativement élevé. Un autre inconvénient réside dans le fait qu'au bout d'un certain temps, ce télescope présente du jeu, tant dans un plan longitudinal que dans un plan transversal, ce qui peut nuire au bon fonctionnement de l'appareil.

L'invention a pour objet un télescope léger, bon marché et facile à manier, auquel il est suspendu un appareil devant être réglé en hauteur. Un tel télescope est en outre au moins aussi robuste que ceux réalisés selon l'art antérieur.

Ces avantages d'un télescope selon l'invention sont obtenus grâce à son agencement, qui permet notamment d'utiliser des tubes légers et des moyens de guidage pratiquement inusables.

Selon l'invention, un télescope à tubes coulissants, pour suspendre un appareil dont la position peut être ajustée par le coulissement l'un dans l'autre des tubes du télescope, est caractérisé en ce que ces tubes sont des tubes profilés étirés qui, à leur surface intérieure sont pourvus de sertissures longitudinales dans lesquelles et serrée une barre en acien trempé sur laquelle roulent des roulements à billes, ces roulements à billes étant montés sur la

2

partie supérieure de la surface du manteau de chaque tube.

A titre d'exemple, d'un caractère nullement limitatif, on trouvera ci-après une description plus détaillée d'une forme d'exécution choisie du télescope conforme à l'invention. Cette description est accompagnée de 4 figures parmis lesquelles :

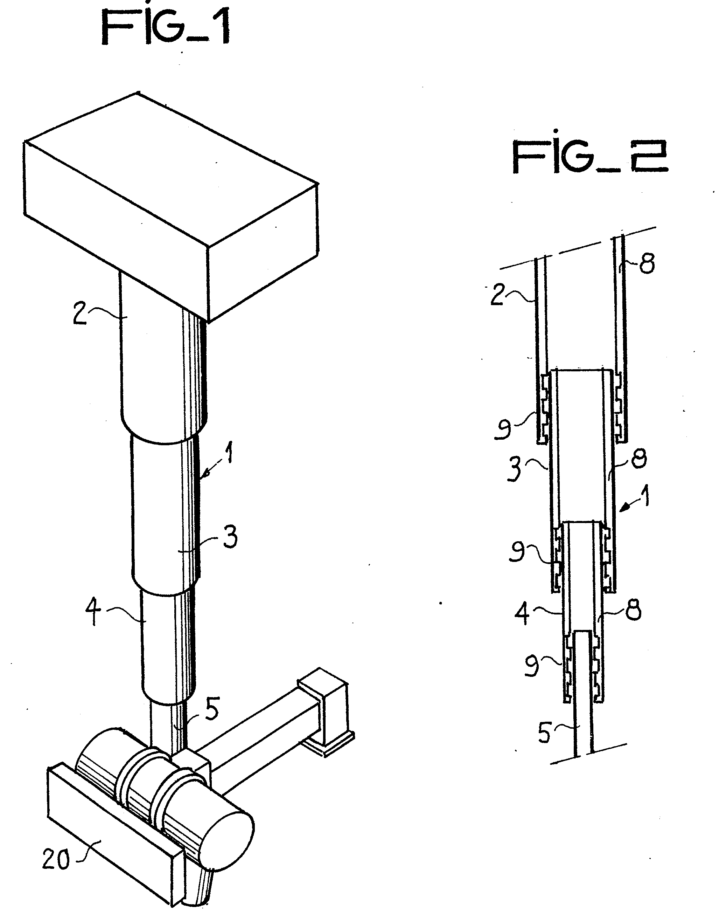

- la figure 1 est une vue en perspective du télescope ;

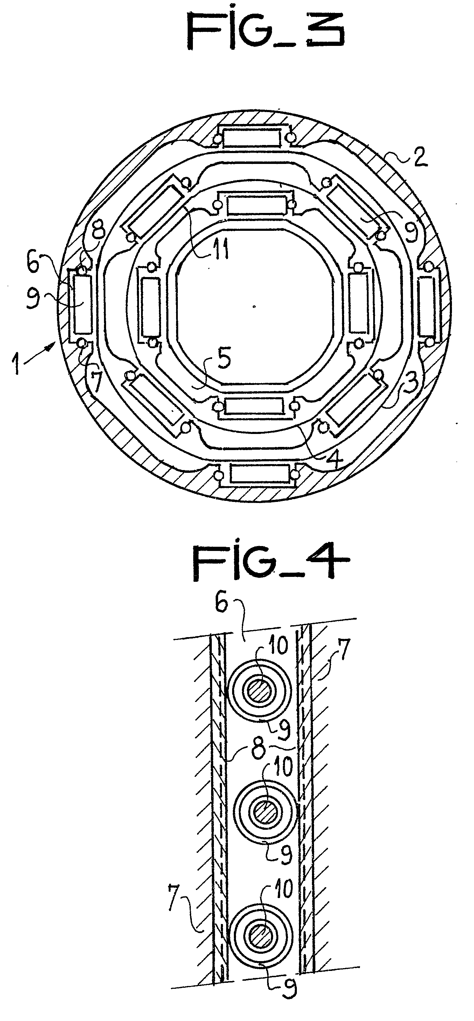

- la figure 2, une coupe longitudinale schématique du télescope ;
- la figure 3, une coupe transversale du télescope ;
- la figure 4, une coupe longitudinale du télescope.

Dans ces figures, on remarque que dans cette forme d'exécution, le télescope 1 portant un porte-tube Röntgen 20 se compose de plusieurs tubes d'aluminuim profilés étirés 2, 3, 4, 5, coulissant l'un dans l'autre, dont les trois tubes extérieurs 2, 3, 4 sont chacun à leur surface intérieure pourvus de quatre rainures longitudinales 6. Chacune des deux surfaces latérales verticales, situées l'une en face de l'autre de chaque rainure, est pourvue d'une sertissure longitudinale 7, dans laquelle est serrée une surface de roulement, formée par une barre 8 en acier trempé et à section transversale circulaire, laquelle barre court sur toute la longueur de chaque tube. Sur la partie supérieure de la surface du manteau de chacun des trois tubes intérieurs 3, 4, 5, il est monté sur un axe 10 diamétralement et l'un en face de l'autre, quatre rangées de roulements à billes 9, placées l'une en dessous de l'autre. Le diamètre de ces roulements à billes 9 est un peu plus faible que la distance entre deux barres 8, tandis que les roulements à billes 9 de chaque rangée sont montés l'un au dessus de l'autre de telle manière que tour à tour un roulement à billes soit en contact avec la barre gauche et la barre droite 8, ces roulements à billes étant noyés dans les rainures 6. On évite ainsi un jeu entre les tubes du télescope, et cela tant dans un plan longitudinal que dans un plan transversal. Les roulements à billes 9 peuvent ainsi courir sans difficultés sur les barres 8, tandis que grâce à la section transversale circulaire de ces barres, il n'existe entre les roulements à billes 9 et les barres 8 qu'un contact linéaire. La résistance de frottement existant entre ces élements

3

est ainsi réduite à un minimum et ainsi, grâce aux tubes prfilés légers, le télescope peut se laisser manier sans peine. En pourvoyant les trois tubes intérieurs 3, 4, 5 de surfaces aplaties 11 tout contre l'extrémité supérieure de la surface de leur manteau, les axes 10 sur lesquels les roulements à billes 9 sont montés, peuvent être facilement fixés aux tubes et raccorder ces tubes de telle manière que l'un soit tout contre l'autre.

Il va de soi que les tubes 2, 3, 4, 5 du télescope 1, coulissant l'un dans l'autre, peuvent être fabriqués en un matériau léger quelconque et qu'ils peuvent avoir une forme et des dimendions quelconques. C'est ainsi que ces tubes pourraient être également en une matière synthétique. Il va également sans dire que les barres 8 en acien trempé pourrraient être remplacées par un fil d'acier ou un autre élément filiforme, comportant une surface de roulement dure pour les roulements à billes 9 et que le nombre de ces roulements à billes, tout comme le nombre de rangées de ces roulements à billes montés sur chaque tube et la disposition de ceux-ci et ce celles-ci, peuvent différer, à condition de rester dans le cadre de l'invention.

4

## REVENDICATIONS

1. Télescopes à tubes coulissants, pour suspendre un appareil (20) dont la position peut être ajustée par le coulissement l'un dans l'autre de tubes (2, 3, 4, 5) du télescope 1, caractérisé en ce que ces tubes (2, 3, 4, 5) sont des tubes profilés étirés qui, à leur surface intérieure sont pouvus de sertissures (7) longitudinales dans lesquelles est serrée une barre (8) en acier trempé sur laquelle roulent des roulements à billes (9), ces roulements à billes (9) étant montés sur la partie supérieure de la surface du manteau de chaque tube (3, 4, 5).

2. Télescope conforme à la revendication 1, caractérisé par le fait que la surface intérieure de chaque tube (2, 3, 4) est pourvue de rainures longitudinales (6) dont les faces latérales verticales, se faisant face, sont pourvues d'une sertissure longitudinale (7), dans laquelle est serrée une barre (8) en acier trempé, rainures (6) précitées dans lesquelles sont noyés des roulements à billes (9) disposés l'un au dessous de l'autre, lesquels roulements à billes courent sur les barres susnommées (9).

3. Télescope conforme à la revendication 3, caractérisé par le fait que les axes (10) de chaque rangée de roulements à billes (9) placés l'un au dessous de l'autre sont montés suivant une ligne brisée, de sorte que tour à tour un roulement à billes (9) coopère avec la barre (8) placée à droite et à gauche dans chaque rainure (6).

4. Télescope conforme à la revendication 2, caractérisé par le fait que les sertissures longitudinales (7) courent sur toute la longueur des tubes (2, 3, 4) et que dans ces sertissures est serree une barre (8) en acier trempé et de section transversale circulaire.

5. Télescope conforme à la revendication 1, caractérisé par le fait que les tubes (2, 3, 4, 5) sont des tubes d'alumunium, profilés et étirés.

FIG_1

FIG_2

0068930

2/2

FIG_3

FIG_4